(19) Europäisches Patentamt
European Patent Office
Office européen des brevets

(11) EP 0 916 343 A1

(12) EUROPÄISCHE PATENTANMELDUNG

(43) Veröffentlichungstag:
**19.05.1999 Patentblatt 1999/20**

(21) Anmeldenummer: **98112091.8**

(22) Anmeldetag: **18.09.1990**

(51) Int. Cl.[6]: **A61K 31/66**
// (A61K31/66, 31:08, 47:08)

(84) Benannte Vertragsstaaten:
**AT BE CH DE DK ES FR GB GR IT LI LU NL SE**

(30) Priorität: **27.09.1989 DE 3932183**

(62) Dokumentnummer(n) der früheren Anmeldung(en) nach Art. 76 EPÜ:
**90117888.9 / 0 419 998**

(71) Anmelder:
**ASTA Medica Aktiengesellschaft**
**01277 Dresden (DE)**

(72) Erfinder:
- **Eibl, Hansjörg**
  **DE 37120 Bovenden-Eddigehausen (DE)**
- **Unger, Clemens**
  **37085 Göttingen (DE)**
- **Engel, Jürgen**
  **63755 Alzenau (DE)**

Bemerkungen:
- Diese Anmeldung ist am 30 - 06 - 1998 als Teilanmeldung zu der unter INID-Kode 62 erwähnten Anmeldung eingereicht worden.
- Geänderte Patentansprüche gemäss Regel 86 (2) EPÜ.

# (54) Verwendung von Alkylphosphorsäure-Verbindungen zur Bekämpfung von Psoriasis-Erkrankungen

(57) Verwendung von Alkylphosphorsäure-Verbindungen der Formel I

$$R - Y - PO_2^{\ominus} - X - R_1 \qquad I$$

wobei in der Formel I
R einen gesättigten oder ungesättigten Kohlenwasserstoffrest mit 12 bis 24 C-Atomen bedeutet oder wobei R die Gruppe $-CH_2-CHR_3-CH_2-Z-R_4$ bedeutet und $R_3$ eine $C_1$-$C_6$-Alkoxygruppe oder ein $C_1$-$C_6$-Alkoxymethylgruppe ist,
Z Sauerstoff oder Schwefel bedeutet und $R_4$ einen $C_1$-$C_{24}$-Alkylrest darstellt,
X ein Sauerstoffatom, NH oder $NR_2$ und Y ein Sauerstoffatom oder NH ist, $R_1$ eine $C_1$-$C_8$-Alkylgruppe ist, oder worin $R_1$ eine $C_2$-$C_8$-Alkylgruppe darstellt, die ungesättigt und/oder mit Halogen, Amino, $C_1$-$C_6$-Alkylamino, Di-$C_1$-$C_6$-alkylamino, Tri-$C_1$-$C_6$-alkylamino, Hydroxy, Carboxy, $C_3$-$C_8$-Cycloalkyl oder Phenyl substituiert ist, für die Herstellung eines Arzneimittels zur Bekämpfung von Psoriasis-Erkrankungen.

EP 0 916 343 A1

## Beschreibung

Alkylphosphorsäure-Verbindungen der Formel I sind bekannte Substanzen, die Antitumorwirkung besitzen.

Es wurde nun gefunden, daß solche Verbindungen der Formel I beziehungsweise deren Salze mit physiologisch verträglichen Säuren auch gegen Psoriasis und hiermit verwandte Erkrankungen wirksam sind.

Die Erfindung betrifft Mittel zur Bekämpfung von Psoriasis-Erkrankungenen wie Psoriasis und psoriasisverwandten Erkrankungen.

Unter Psoriasis-Erkrankungen werden im Sinne der Erfindung Hauterkrankungen verstanden, die mit Hyperkeratosen einhergehen wie insbesondere Psoriasis, Arthropathia psoriatica, Parapsoriasis-Erkrankungen.

Zur Behandlung von Psoriasis werden zur Zeit hauptsächlich Corticosteroide eingesetzt. In schweren Fällen von Psoriasis werden sogar Zytostatika (zum Beispiel Methotrexat) verwendet. Alle diese Substanzen besitzen jedoch starke und schwerwiegende Nebenwirkungen.

Demgegenüber zeigen die Verbindungen der Formel I in den angewendeten Dosierungen keine beziehungsweise zu vernachlässigende Nebenwirkungen. Beispielsweise ist die Wirkung der topischen Therapie unter Verwendung von Verbindungen der Formel I derjenigen der üblichen Dermatika, die bei Psoriasis zur Anwendung kommen (Keratolytika und Kortikoide), deutlich überlegen.

Die pharmazeutischen Zubereitungen für die Anwendung bei Psoriasis-Erkrankungen und hiermit verwandten Erkrankungen enthalten im allgemeinen zwischen 1 bis 1000 vorzugsweise 5 bis 500 mg insbesondere 10 bis 100 mg pro Einzeldosis an Komponente I (Komponente I = Summe der Gewichtsmengen der Einzelverbindungen der Formel I) bei oraler, parenteraler, rektaler, vaginaler oder inhalativer Applikation.

Falls eine Zubereitung 2 oder mehr Einzelverbindungen der Formel I enthält, gelten solche Zahlen stets für die Gesamtmenge an der Komponente I. Dies gilt analog für die folgenden Mengenangaben, die sich auf die Komponente I (Komponente I = Summe der Einzelverbindungen der Formel I) im Zusammenhang mit Dosierungsangaben und ihrer Gewichtsmenge in den entsprechenden pharmazeutischen Zubereitungen beziehen sowie entsprechende Mengenangaben in den Ansprüchen. Unter Verbindung I wird jeweils die durch die Formel I ausgedrückte Einzelsubstanz oder deren physiologisch verträgliches Salz verstanden.

Bei Zubereitungen zur lokalen Applikation auf die Haut und Schleimhäute beträgt die Menge an der Komponente I zum Beispiel zwischen 0,1 mg und 1000 mg vorzugsweise 0,1 bis 500 mg.

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen. Als flüssige Anwendungsformen kommen zum Beispiel in Frage: Ölige oder alkoholische beziehungsweise wässrige Lösungen sowie Suspensionen und Emulsionen. Bevorzugte Anwendungsformen sind Tabletten, die zwischen 10 und 100 mg oder Lösungen, die zwischen 0,01 bis 10 Gewichtsprozent der Komponente I enthalten.

Die Einzeldosis an der Komponente I (das heißt Gesamtmenge an Verbindungen I) kann beispielsweise liegen

a) bei oralen Arzneiformen zwischen 1 - 1000 mg, vorzugswiese 10 - 500 mg;

b) bei parenteralen Arzneiformen (zum Beispiel intravenös, intramuskulär) zwischen 1 - 1000 mg, vorzugsweise 10 - 500 mg;

c) bei Arzneiformen zur Inhalation (Lösungen oder Aerosole) zwischen 1 - 1000 mg; vorzugsweise 10 - 500 mg;

d) bei Arzneiformen zur rektalen oder vaginalen Applikation zwischen 1 - 1000 mg, vorzugsweise 10 - 500 mg;

e) bei Arzneiformen zur lokalen Applikation auf die Haut und Schleimhäute (zum Beispiel in Form von Lösungen, Lotionen, Emulsionen, Salben und so weiter) zwischen 0,1 mg - 1000 mg, vorzugsweise 0,1 - 500 mg wobei die Konzentration an der Komponente I (das heißt Gesamtmenge an Verbindungen I) in solchen Zubereitungen zum Beispiel 0,01 bis 12, insbesondere 0,1 - 8 Gewichts% ist.

Beispielsweise können 3mal täglich 1 bis 3 Tabletten mit einem Gehalt von 1 bis 100 mg Komponente I (das heißt Gesamtmenge I) oder zum Beispiel bei intravenöser Injektion 1 bis 3mal täglich eine Ampulle von 1 bis 10 ml Inhalt mit 1 bis 100 mg Komponente I (das heißt Gesamtmenge I) empfohlen werden. Bei oraler Verabreichung ist die minimale tägliche Dosis beispielsweise 1 mg; die maximale tägliche Dosis bei oraler Verabreichung soll nicht über 1000 mg liegen.

Für die Behandlung von Hunden und Katzen liegt die orale Einzeldosis der Komponente I im allgemeinen zwischen ungefähr 0,01 und 60 mg/kg Körpergewicht; die porenterale Dosis ungefähr zwischen 0,01 und 60 mg/kg Körpergewicht.

Für die Behandlung von Pferden und Vieh liegt die orale Einzeldosis der Komponente I im allgemeinen zwischen ungefähr 0,05 und 100 mg/kg; die parenterale Einzeldosis ungefähr zwischen 0,05 und 100 mg/kg Körpergewicht.

Die akute Toxizität der Verbindungen I an der Maus (ausgedrückt durch die LD 50 mg/kg; Methode nach Miller und Tainter: Proc. Soc. Exper. Biol. a. Med. 57 (1944) 261) liegt beispielsweise bei oraler Applikation zwischen 300 und 1000 mg/kg.

Die erfindungsgemäßen Arzneimittel sind dadurch gekennzeichnet, daß sie als Wirkstoff wenigstens eine Verbindung der allgemeinen Formel I gemäß dem Anspruch 1

$$R - Y - PO_2^{\theta} - X - R_1$$

oder ein physiologisch verträgliches Salz hiervon, gegebenenfalls zusammen mit üblichen pharmakologischen Zusatz- und Verdünnungsmitteln enhalten. Vorzugsweise kommen als Wirkstoffe in Frage: Hexadecylphosphocholin, Oleylphosphocholin, Hexadecylphosphorsäure-(N,N)-bis-(2-chlorethyl)-amid.

Die Formel I umfaßt auch die möglichen Enantiomere und Diastereomere. Falls die Verbindungen Racemate sind, können diese in an sich bekannter Weise, zum Beispiel mittels einer optisch aktiven Säure, in die optisch aktiven Isomere gespalten werden. Bevorzugt werden jedoch von vornherein enantiomere oder gegebenenfalls diastereomere Ausgangsstoffe eingesetzt, wobei dann als Endprodukt eine entsprechende reine optisch aktive beziehungsweise diastereomere Verbindung erhalten wird.

Im Rahmen der Erfindung ist R vorzugsweise eine Alkylgruppe der angegebenen Kettenlänge, die mit dem Sauerstoff des Glykolrestes über ein endständiges C-Atom oder auch über ein C-Atom innerhalb der Alkylkette verknüpft ist (zum Beispiel über das C-Atom 2 oder C-Atom 3 oder ein anderes mittleres C-Atom). Diese Alkylkette kann geradkettig oder verzweigt sein. Die Alkylkette R kann eine, zwei oder drei Kohlenstoff-Doppelbindungen oder Dreifachbindungen, die auch gemischt vorliegen können, enthalten und/oder Halogensubstituenten aufweisen. Als Halogenatome kommen in Frage: Fluor, Chlor oder Brom. In der Kette R können ein bis drei solcher Halogenatome vorliegen, wobei diese sich an einem oder an verschiedenen C-Atomen des Restes R befinden können.

Bevorzugt werden neben den gesättigten geradkettigen Alkylresten solche mit ein oder zwei Kohlenstoffdoppelbindungen im Molekül. Besonders bevorzugt werden solche Substituenten R, welche einen Alkylrest mit 14 bis 20, vorzugsweise 15 bis 20, insbesondere 16 bis 20 C-Atomen oder einen entsprechenden Alkenylrest mit 14 bis 20, vorzugsweise 15 bis 20, insbesondere 16 bis 20 C-Atomen enthalten.

Beispiele für halogensubstituierte Reste R sind:
Chlorhexadecyl, Bromhexadecyl, Fluorhexadecyl, 9,10-Dibromoctadecyl, 2,3-Dibromoctadecyl, 15,16-Dibromhexadecyl, Bromtetradecyl.

Beispiele für ungesättigte Reste R sind:
9-Octadecenylrest (Oleylalkoholrest, insbesondere bedeutet in der Formel I R diesen 9-Octadecenylrest), 15-Hexadecenylrest, 9,12-Octadecadienylrest (Linoleylrest).

Falls mehr als eine Doppel- oder Dreifachbindung vorliegt, sind diese konjungiert.
Beispiele für gesättigte und unsubstituierte Reste R sind: Tetradecylrest, Hexadecylrest, Octadecylrest.

R bedeutet zum Beispiel vorzugsweise die Gruppe $-CH_2-CHR_3-CH_2-Z-R_4$, wenn Y und X Sauerstoff sind und $R_1$ eine $C_1$-$C_6$-Trialkylaminogruppe (insbesondere Trimethylamino) ist, die mit X über eine $C_2$-$C_3$-Alkylkette verknüpft ist und wobei die positive Ladung des Trialkylaminkations durch das Phosphorsäureanion neutralisiert wird.
Bei den Alkoxygruppen $R_3$ und $R_4$ handelt es sich vorzugsweise um Methoxygruppen. Der $C_1$-$C_{24}$-Alkylrest $R_4$ besteht vorzugsweise aus 10 bis 20, insbesondere 12 bis 18 C-Atomen und ist vorzugsweise unverzweigt.

Falls $R_1$ beziehungsweise $R_2$ eine unsubstituierte Alkylgruppe bedeuten, besteht diese zum Beispiel aus 1 - 6, vorzugsweise 1 - 4 C-Atomen. Falls $R_1$ beziehungsweise $R_2$ eine ungesättigte Alkylgruppe bedeutet, besteht sie insbesondere aus 3 bis 6-C-Atomen, wobei zwischen der ungesättigten Funktion einer solchen ungesättigten Alkylgruppe und X mindestens eine einfache C-C-Bindung vorliegen muß. Insbesondere handelt es sich um $C_3$-$C_6$-Alkenylgruppen. Beispiele hierfür sind: Allyl, Butenyl, Pentenyl, Hexenyl.

Falls $R_1$ beziehungsweise $R_2$ substituiert sind, handelt es sich insbesondere um geradkettige Alkyl- oder Alkenylreste, vorzugsweise besteht $R_1$ in diesem Fall aus 2 - 6 C-Atomen, wobei die angegebenen Substituenten vorzugsweise in der ω-Stellung der Alkyl- beziehungsweise Alkenylgruppe $R_1$ beziehungsweise $R_2$ stehen; beispielsweise handelt es sich um den Ethyl- oder geraden Propylrest mit einem der angegebenen Substituenten in ω-Stellung (das heißt in 2-Stellung bei Ethyl und 3-Stellung bei Propyl).
Falls $R_1$ ein 2-tert.-Butyloxycarbonylaminoethyl-Rest oder ein 2-tert.-Butyloxycarbonylethyl-Rest ist, handelt es sich vorzugsweise um die D- oder L-Form.

Unter den Substituenten von $R_1$ werden die Trialkylammoniumethylreste bevorzugt, insbesondere wenn X ein Sauerstoffatom ist, wobei die Trialkylreste vorzugsweise jeweils aus einem, zwei oder drei C-Atomen bestehen, vorzugsweise handelt es sich um Methylgruppen. Besonders bevorzugt ist daher der Trimethylammonium-ethylrest. In dieser besonders bevorzugten Ausführungsform handelt es sich bei den Verbindungen der Formel I um

Phosphatidylcholinderivate.

Im Falle des $C_3$-$C_8$-Cycloalkyl-Substituenten besteht dieser insbesondere aus 3 - 6 C-Atomen (zum Beispiel Cyclopropyl bis Cyclohexyl). Bei der 2,3-Dihydroxypropyl-(1)-gruppe handelt es sich insbesondere um die sn-1,2-dihydroxy-propylamino-(3)-Struktur oder um die sn-2,3-dihydroxy-propylamino-(1)-Struktur.

Weitere Beispiele für bevorzugte Verbindungen der Formel I sind:
Oleyl-phospho-(N,N,N-trimethyl)-propanolamin, Oleyl-phospho-(N,N,N-trimethyl)-butanolamin, Oleyl-phospho-(N,N,N-trimethyl)-pentanolamin, Oleyl-phosphoserin, Oleyl-phosphoethanolamin, Oleyl-phosphopropanolamin, Oleyl-phosphobutanolamin, Oleyl-phosphoglycerin, Hexadecyl-phospho-(N,N,N-trimethyl)-propanolamin, 1-Octadecyl-2-methyl-sn-glycero-3-phosphocholin (zum Beispiel ET-18-$OCH_3$, siehe Deutsches Patent 26 19 686). 1-Hexadecylmercapto-2-methoxymethyl-propanol-3-phosphocholin (Ilmofosin)

Als Salze kommen innere Salze in Frage (zum Beispiel wenn $R_1$ eine Trimethylammonio-Alkylgruppe bedeutet) oder Salze mit physiologisch verträglichen Kationen. Die erfindungsgemäßen Arzneimittel beziehungsweise die Verbindungen I können als innere Salze vorliegen, zum Beispiel wenn $R_1$ eine Aminogruppe enthält. Falls keine inneren Salze vorliegen, beziehungsweise der Rest $R_1$ keine basische Gruppe enthält, wird die negative Ladung der Phosphorsäuregruppe durch ein physiologisch verträgliches Kation abgesättigt. Als solche physiologisch verträglichen Kationen kommen zum Beispiel in Frage: Alkalikationen (Na, K), Erdalkalikationen (Mg, Ca) oder die Kationen von organischen Aminen, wie zum Beispiel Guanidinium-, Morpholinium, Cyclohexylammoniumkation, Ethylendiammoniumkation, Piperazoniumkation (in beiden letzteren Fällen ein- oder zweibasisch) oder das Kation, welches sich von einem Amin der Formel $NR_aR_bR_c$ ableitet, worin die Reste $R_a$ bis $R_c$ gleich oder verschieden sind und Wasserstoff, $C_1$-$C_2$-Alkylgruppen oder Oxyethylgruppen bedeuten.
Falls es sich um Kationen handelt, welche sich von einem Amin der Formel $NR_aR_bR_c$ ableiten, handelt es sich vorzugsweise um das Ammoniumkation oder um ein mit ein bis drei $C_1$-$C_2$-Alkylgruppen substituiertes Ammoniumkation oder um ein mit ein bis drei 2-Hydroxyethylgruppen substituiertes Ammoniumkation.

Die negative Ladung der Verbindungen der Formel I wird also beispielsweise durch eine im Molekül vorhandene basische Aminogruppe oder eine niedrigmolekulare Mono-, Di- oder Tri-$C_1$-$C_6$-alkylaminogruppe oder durch ein zusätzliches physiologisch verträgliches Kation abgesättigt.

Die Herstellung der Wirkstoffe gemäß der allgemeinen Formel I ist grundsätzlich bekannt und kann zum Beispiel nach an sich bekannten Methoden beziehungsweise analog hierzu erfolgen. Das Grundgerüst läßt sich leicht durch Umsetzung einer Verbindung der Formel ROH oder einem funktionellen Derivat davon mit Phosphoroxychlorid und Triethylamin, Umsetzung des Produktes mit einer Verbindung $HXR_1$ und saure Spaltung, wobei R, $R_1$ und X die obige Bedeutung besitzen, erhalten.

Die erfindungsgemäßen pharmazeutischen Zusammensetzungen beziehungsweise Arzneimittel enthalten als Wirkstoff mindestens eine Verbindung (Komponente) der Formel I gegebenenfalls in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche Stoffe in Frage, die in folgenden Literaturstellen als Hilfsstoffe für Pharmazie, Kosmetik und angrenzende Gebiete empfohlen beziehungsweise angegeben sind: Ullmanns Encyklopädie der technischen Chemie, Band 4 (1953), Seite 1 bis 39; Journal of Pharmaceutical Sciences, Band 52 (1963), Seite 918 und ff.; H.v.Czetsch-Lindenwald, Hilfsstoffe für Pharmazie und angrenzende Gebiete; Pharm. Ind., Heft 2 (1961), Seite 72 und ff.; Dr. H.P. Fiedler, Lexikon der Hilfsstoffe für Pharmazie, Kosmetik und angrenzende Gebiete Cantor KG. Aulendorf in Württemberg 1981.

Beispiele hierfür sind Gelatine, natürliche Zucker wie Rohrzucker oder Milchzucker, Lecithin, Pektin, Stärke (zum Beispiel Maisstärke), Cyclodextrine und Cyclodextrinderivate, Polyvinylpyrrolidon, Polyvinylacetat, Gelatine, Gummi arabicum, Alginsäure, Tylose, Talkum, Lycopodium, Kieselsäure (zum Beispiel kolloidale), Cellulose, Cellulosederivate (zum Beispiel Celluloseether, bei denen die Cellulose-Hydroxygruppen teilweise mit niederen gesättigten aliphatischen Alkoholen und/oder niederen gesättigten aliphatischen Oxyalkoholen verethert sind, zum Beispiel Methyloxypropylcellulose, Methylcellulose, Hydroxypropylmethylcellulose, Hydroxypropylmethylcellulosephthalat); Fettsäuren sowie Magnesium-, Calcium- oder Aluminiumsalze von Fettsäuren mit 12 bis 22 C-Atomen, insbesondere der gesättigten (zum Beispiel Stearate), Emulgatoren, Öle und Fette, insbesondere pflanzliche (zum Beispiel Erdnußöl, Rizinusöl, Olivenöl, Sesamöl, Baumwollsaatöl, Maisöl, Weizenkeimöl, Sonnenblumensamenöl, Kabeljau-Leberöl, jeweils auch hydriert; Mono-, Di- und Triglyceride von gesättigten Fettsäuren $C_{12}H_{24}O_2$ bis $C_{18}H_{36}O_2$ und deren Gemische, pharmazeutisch verträgliche ein- oder mehrwertige Alkohole und Polyglykole wie Polyethylenglykole sowie Derivate hiervon, Ester von aliphatischen gesättigten oder ungesättigten Fettsäuren (2 bis 22 C-Atome, insbesondere 10 - 18 C-Atome) mit einwertigen aliphatischen Alkoholen (1 bis 20 C-Atome) oder mehrwertigen Alkoholen wie Glykolen, Glycerin, Diethylenglykol, Pentaerythrit, Sorbit, Mannit und so weiter, die gegebenenfalls auch verethert sein können, Ester der Zitronensäure mit primären Alkoholen, Essigsäure, Benzylbenzoat, Dioxolane, Glyzerinformale, Tetrahydrofurfurylalkohol, Polyglykolether mit $C_1$-$C_{12}$-Alkoholen, Dimethylacetamid, Lactamide, Lactate, Ethylcarbonate, Silicone

(insbesondere mittelviskose Polydimethylsiloxane), Calciumcarbonat, Natriumcarbonat, Calciumphosphat, Natriumphosphat, Magnesiumcarbonat und ähnliche.

Als weitere Hilfsstoffe kommen auch Stoffe in Frage, die den Zerfall bewirken (sogenannte Sprengmittel) wie: quervernetztes Polyvinylpyrrolidon, Natriumcarboxymethylstärke, Natriumcarboxymethylcellulose oder mikrokristalline Cellulose. Ebenfalls können bekannte Hüllstoffe verwendet werden. Als solche kommen beispielsweise in Frage: Polymerisate sowie Copolymerisate der Acrylsäure und/oder Methacrylsäure und/oder deren Ester; Copolymerisate aus Acryl- und Methacrylsäureestern mit einem geringen Gehalt an Ammoniumgruppen (zum Beispiel Eudragit® RS), Copolymerisate aus Acryl- und Methacrylsäureestern und Trimethylammoniummethacrylat (zum Beispiel Eudragit® RL); Polyvinylacetat; Fette, Öle, Wachse, Fettalkohole; Hydroxypropylmethylcellulosephthalat oder -acetatsuccinat; Cellulose-, Stärke- sowie Polyvinylacetatphthalat; Carboxymethylcellulose; Methylcellulose-phthalat, -succinat, -phthalatsuccinat sowie -phthalatsäurehalbester; Zein; Ethylcellulose sowie -succinat; Schellack, Gluten; Ethylcarboxyethylcellulose; Ethacrylat-Maleinsäureanhydrid-Copolymer; Maleinsäureanhydrid-Vinylmethylether-Copolymer; Styrol-Maleinsäure-Copolymerisate; 2-Ethyl-hexylacrylatmaleinsäureanhydrid; Crotonsäure-Vinylacetat-Copolymer; Glutaminsäure/Glutaminsäureester-Copolymer; Carboxymethylethyl-celluloseglycerinmonooctanoat; Celluloseacetatsuccinat; Polyarginin.

Als Plastifizierungsmittel für Hüllstoffe können in Frage kommen:

Citronen- und Weinsäureester (Acetyltriethyl-, Acetyltributyl-, Tributyl-, Triethyl-citrat);
Glycerin und Glycerinester (Glycerindiacetat, -triacetat, acetylierte Monoglyceride, Rizinusöl);
Phthalsäureester (Dibutyl-, Diamyl-, Diethyl-, Dimethyl-, Dipropyl-phthalat), D-(2-Methoxy- oder ethoxyethyl)-phthalat, Ethylphthalyl-, Butylphthalylethyl- und Butylglycolat; Alkohole (Propylenglycol, Polyethylenglycol verschiedener Kettenlängen), Adipate (Diethyl-adipat, Di(2-Methoxy- oder ethoxyethyladipat);
Benzophenon; Diethyl- und Dibutylsebacat, -succinat, -tartrat; Diethylenglycoldipropionat; Ethylenglykoldiacetat, -dibutyrat, -dipropionat; Tributylphosphat, Tributyrin; Polyethylenglykolsorbitanmonooleat (Polysorbate wie Polysorbat 80); Sorbitanmonooleat.

Zur Herstellung von Lösungen oder Suspensionen kommen beispielsweise Wasser oder physiologisch verträgliche organische Lösungsmittel in Frage, wie zum Beispiel Ethanol, Propanol, Isopropanol, 1,2-Propylenglykol, Glycerin-$C_1$-$C_{12}$-alkylether, insbesondere 1-Glycerin-$C_1$-$C_9$-alkylether wie zum Beispiel Glycerin-1-n-propylether, Glycerin-1-n-hexylether, Glycerin-1-n-nonylether Polyglykole und deren Derivate, Dimethylsulfoxid, Fettalkohole, Triglyceride, Partialester des Glycerins, Paraffine und ähnliche.
Für injizierbare Lösungen oder Suspensionen kommen zum Beispiel nicht-toxische parenteral verträgliche Verdünnungsmittel oder Lösungsmittel in Frage, wie zum Beispiel: Wasser, 1,3-Butandiol, Ethanol, 1,2-Propylenglykol, Polyglykole in Mischung mit Wasser, Ringer's Lösung, isotonische Kochsalzlösung oder auch gehärtete Öle einschließlich synthetischer Mono- oder Diglyceride oder Fettsäuren wie Oleinsäure.

Bei der Herstellung der Zubereitungen können bekannte und übliche Lösungsvermittler, beziehungsweise Emulgatoren, verwendet werden. Als Lösungsvermittler und Emulgatoren kommen beispielsweise in Frage:
Polyvinylpyrrolidon, Sorbitanfettsäureester wie Sorbitantrioleat, Phosphatide, wie Lecithin, Acacia, Traganth, polyoxyethyliertes Sorbitanmonooleat und andere ethoxylierte Fettsäureester des Sorbitan, polyoxyethylierte Fette, polyoxyethylierte Oleotriglyceride, linolisierte Oleotriglyceride, Polyethylenoxyd-Kondensationsprodukte von Fettalkoholen, Alkylphenolen oder Fettsäuren oder auch 1-Methyl-3-(2-hydroxyethyl)-imidazolidon-(2). Polyoxyethyliert bedeutet hierbei, daß die betreffenden Stoffe Polyoxyethylenketten enthalten, deren Polymerisationsgrad im allgemeinen zwischen 2 bis 40 und insbesondere zwischen 10 bis 20 liegt.
Solche polyoxyethylierten Stoffe können beispielsweise durch Umsetzung von hydroxylgruppenhaltigen Verbindungen (beispielsweise Mono- oder Diglyceride oder ungesättigte Verbindungen wie zum Beispiel solchen, die Ölsäurereste enthalten) mit Ethylenoxyd erhalten werden (zum Beispiel 40 Mol Ethylenoxyd pro Mol Glycerid).
Beispiele für Oleotriglyceride sind Olivenöl, Erdnußöl, Rizinusöl, Sesamöl, Baumwollsaatöl, Maisöl.
Siehe auch Dr. H. P. Fiedler "Lexikon der Hilfsstoffe für Pharmazie, Kosmetik und angrenzende Gebiete" 1971, S. 191-195.

Darüberhinaus ist der Zusatz von Konservierungsmitteln, Stabilisatoren, Puffersubstanzen, zum Beispiel Calciumhydrogenphosphat, kolloidales Aluminiumhydroxyd, Geschmackskorrigentien, Süßmitteln, Farbstoffen, Antioxydantien und Komplexbildnern (zum Beispiel Ethylendiaminotetraessigsäure) und dergleichen möglich.
Gegebenenfalls ist zur Stabilisierung des Wirkstoffmoleküls mit physiologisch verträglichen Säuren oder Puffern auf einen pH-Bereich von ca. 3 bis 7 einzustellen Im allgemeinen wird ein möglichst neutraler bis schwach saurer (bis pH 5) pH-Wert bevorzugt.

Zur Herstellung von dermal anzuwendenden Zubereitungen kommen in Frage die vorgenannten Substanzen und streichfähige oder flüssige Kohlenwasserstoffe wie Vaselin oder Paraffin oder Gele aus Paraffinkohlenwasserstoffen und Polyethylen, Fette und Öle pflanzlichen oder tierischen Ursprungs, die zum Teil auch hydriert sein können oder

synthetische Fette wie Glyceride der Fettsäuren $C_8$-$C_{18}$, sodann Bienenwachs, Cetylpalmitat, Wollwachs, Wollwachsalkohole; Fettalkohole wie Cetylalkohol, Stearylalkohol, Polyethylenglykole vom Molekulargewicht 200 bis 20 000; flüssige Wachse wie Isopropylmyristat, Isopropylstearat, Äthyloleat; Emulgatoren wie Natrium-, Kalium-, Ammoniumsalze der Stearinsäure oder Palmitinsäure sowie Triethanolaminstearat, Alkalisalze der Ölsäure, Ricinolsäure, Salze von sulfurierten Fettalkoholen wie Natriumlaurylsulfat, Natriumcetylsulfat, Natriumstearylsulfat, Salze der Gallensäure, Sterole wie Cholesterol, Partialfettsäureester mehrwertiger Alkohole wie Ethylenglykolmonostearat, Glycerolmonostearat, Pentaerythritmonostearat, Partialfettsäureester des Sorbitans, Partialfettsäureester des Polyoxyethylensorbitans, Sorbitolether des Polyoxyethylens, Fettsäureester des Polyoxyethylens, Fettalkoholether des Polyoxyethylens, Fettsäureester der Saccharose, Fettsäureester des Polyglycerols, Lecithin.

Als Antioxydantien kommen beispielsweise Natriummetabisulfit, Ascorbinsäure, Gallussäure, Gallussäurealkylester, Butylhydroxyanisol, Nordihydroguajaretsäure, Tocopherole sowie Tocopherole + Synergisten (Stoffe, die Schwermetalle durch Komplexbildung binden, beispielsweise Lecithin, Ascorbinsäure, Phosphorsäure) zur Anwendung. Der Zusatz der Synergisten steigert die antioxygene Wirkung der Tocopherole erheblich.
Als Konservierungsmittel kommen beispielsweise Sorbinsäure, p-Hydroxybenzoesäureester (zum Beispiel Niederalkylester), Benzoesäure, Natriumbenzoat, Trichlorisobutylalkohol, Phenol, Kresol, Benzethoniumchlorid und Formalinderivate in Betracht.

Die pharmazeutische und galenische Handhabung der Verbindungen der Formel I erfolgt nach den üblichen Standardmethoden. Beispielsweise werden Wirkstoff(e) und Hilfs- beziehungsweise Trägerstoffe durch Rühren oder Homogenisieren (zum Beispiel mittels üblicher Mischgeräte) gut vermischt, wobei im allgemeinen bei Temperaturen zwischen 20 und 80° C, vorzugsweise 20 bis 50° C, insbesondere bei Raumtemperatur gearbeitet wird. In übrigen wird auf das folgende Standardwerk verwiesen: Sucker, Fuchs, Speiser, Pharmazeutische Technologie, Thieme-Verlag Stuttgart, 1978.

Die Applikation kann auf die Haut oder Schleimhaut oder in das Körperinnere erfolgen, beispielsweise oral, enteral, pulmonal, rectal, nasal, vaginal, lingual, intravenös, intraarteriell, intrakardial, intramuskulär, intraperitoneal, intracutan, subcutan.
Bei den parenteralen Zubereitungsformen handelt es sich insbesondere um sterile beziehungsweise sterilisierte Erzeugnisse.
Die Konzentration an der Verbindung I (Komponente I, das heißt Gesamtmenge) in den Arzneiformen zur lokalen Applikation beträgt 0,01 bis 12, vorzugsweise 0,05 bis 10, insbesondere 0,1 bis 8 oder auch 0,5 bis 6 Gewichts%.

Zur Behandlung der Psoriasis werden die Verbindungen I (Komponente I) insbesondere lokal, zum Beispiel in Form von Lösungen, Tinkturen, Suspensionen, Emulsionen, Salben, Gelen, Cremes, Pasten, Lotionen oder Shampoos eingesetzt. Bevorzugt werden wasserfreie Zubereitungen verwendet, wodurch die gleichzeitige Anwendung von Salicylsäure und/oder Harnstoff ermöglicht wird. Derartige Zubereitungen, die durch den Zusatz von Tensiden auch abwaschbar gemacht werden können, sind beispielsweise in der Deutschen Offenlegungsschrift 36 03 859 beschrieben. Der Harnstoff kann entweder als Tensid-Harnstoff-Einschlußverbindung oder auch in freier Form vorliegen. Zubereitungen, die weder Harnstoff noch Salicylsäure enthalten, können selbstverständlich auch Verwendung finden.

Die Einsatzkonzentrationen an der Verbindung I (jeweils Gesamtmenge) betragen hierbei beispielsweise 0,1 bis 10 % (Gewicht/Gewicht), vorzugsweise 0,5 bis 8 %, insbesondere 1 bis 6 %. Die Einsatzkonzentrationen an Salicylsäure betragen beispielsweise 0,1 bis 10 %, vorzugsweise 0,2 bis 8 %, insbesondere 0,5 bis 5 %. Die Einsatzkonzentrationen an Harnstoff betragen beispielsweise 1 bis 20 %, vorzugsweise 3 bis 18 %, insbesondere 5 bis 15 %.

Für die topische Applikation, hat es sich beispielsweise als günstig herausgestellt, die Verbindungen I (Komponente I) zusammen mit wenigstens einem Alkylglycerin mit 2 bis 12 Kohlenstoffatomen im Alkylrest, der in Form einer Ethergruppe an eine der primären oder sekundären OH-Gruppen des Glycerins gebunden vorliegen kann, einzusetzen. Derartige Alkylglycerine steigern beziehungsweise verbessern die Wirkung der Verbindungen I. Bevorzugt werden hierbei Alkylglycerine mit 3 bis 9 C-Atomen allein oder in Mischung verwendet.

Besonders günstige Wirkungen besitzt daher ein Arzneimittel, welches

a) eine oder mehrere Verbindungen der Formel I (Komponente I) und

b) ein Alkylglycerin der allgemeinen Formel II

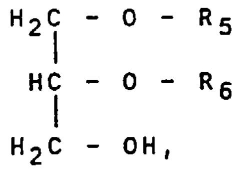

in welcher einer der Reste $R_5$ und $R_6$ eine Alkylgruppe mit 2 bis 12 C-Atomen und der andere Rest ein H-Atom bedeutet, enthält,
sowie gegebenenfalls weitere übliche pharmakologische Zusatz- und Verdünnungsmittel.

Vorzugsweise kommt ein Gemisch aus Wasser und einer Alkylglycerin-Mischung von Nonyl- beziehungsweise Octylglycerin, Hexyl- beziehungsweise Pentylglycerin und Propyl- beziehungsweise Ethylglycerin in Frage. Beispielsweise enthält eine entsprechende Formulierung für die topische Anwendung 1 bis 100 mg Verbinding I (Komponente I, das heißt Gesamtmenge I) pro ml Alkylglycerin der Formel II beziehungsweise einer entsprechenden Alkylglycerin-Mischung mit Wasser.

Eine derartige Mischung wird im folgenden auch als Kaskade bezeichnet.
Der Gehalt an Komponente I in mg/ml Kaskade wird durch einen nachgesetzten Index bezeichnet, derart, daß zum Beispiel ein Kaskadengemisch, welches 10 mg/ml Komponente I enthält, als Kaskade 10 ein Gemisch mit 60 mg Komponente I pro ml Kaskade als Kaskade 60 bezeichnet wird.

Die Herstellung der Alkylglycerine ist bekannt, beispielsweise aus der DE-OS 33 43 530.8.
Beispielsweise werden Alkylglycerin-Wasser-Mischungen, welche zum Beispiel Nonylglycerin, Octylglycerin, Hexylglycerin, Pentylglycerin, Propylglycerin und Ethylglycerin enthalten, bevorzugt. Vorzugsweise enthalten solche wäßrigen Mischungen 3 der genannten Glycerinether, und zwar einen niederen (Ethyl, Propyl), einen mittleren (Pentyl, Hexyl) und einen höheren (Octyl, Nonyl), wobei die Gewichtsmenge an dem niederen Ether etwa so groß ist wie die Summe der Gewichtsmengen an den bei den anderen Glycerinethern. Die Wassermenge ist etwa gleich der Menge an dem niederen Glycerinether und beträgt beispielsweise die Hälfte der Gesamtmenge an den vorliegenden Glycerinethern. Beispiele für solche Glycerinether-Wasser-Mischungen sind im folgenden angeführt:

| | Wasser | | Glycerin-Propyl-ether | | Glycerin-Hexyl-ether | | Glycerin-Nonyl-ether |
|---|---|---|---|---|---|---|---|
| Gewichts-teile | 2 | : | 2 | : | 1 | : | 1 |

| | Wasser | | Glycerin-Ethyl-ether | | Glycerin-Pentyl-ether | | Glycerin-Octyl-ether |
|---|---|---|---|---|---|---|---|
| Gewichts-teile | 2 | : | 2 | : | 1 | : | 1 |

Zur topischen Applikation sind die Arzneimittel mit den Alkylglycerinen der Formel II in besonderem Maße

geeignet. Um beispielsweise Psoriasis und hiermit verwandte Erkrankungen zu behandeln, werden die betroffenen Hautbezirke beispielsweise mit Kaskade 10 bis Kaskade 80 zwei- bis dreimal täglich eingerieben. Schädliche Nebenwirkungen konnten bisher nicht beobachtet werden.

Die Zubereitungsart der Verbindungen I (Komponente I) in Form der Kaskade (zum Beispiel in Form der Lösungen Kaskade 10 bis Kaskade 100, insbesondere Kaskade 40 bis 60) eignet sich auch gut für die Herstellung von Suppositorien für die rektale Einführung. Auch hiermit läßt sich Psoriasis beziehungsweise Psoriasis-Erkrankungen gut behandeln.

Eine besonders günstige Trägermischung für die Komponente I besteht aus einer Mischung von etwa 4 Gewichtsteilen Wasser, 4 Gewichtsteilen Propylglycerin und je 2 Gewichtsteilen Hexylglycerin und Nonylglycerin.

Zur Herstellung der Arzneimittel, die die Komponente I in Gegenwart von einem Glycerinether der Formel II oder einer Mischung von solchen Glycerinethern der Formel II enthalten, wird die Komponente I beispielsweise mit 10 000 bis 7, insbesondere 100 bis 10, vorzugsweise 30 bis 16 Gewichtstellen (bezogen jeweils auf einen Gewichtsteil) von mindestens einem Glycerinether der Formel II oder einem Gemisch solcher Glycerinether sowie gegebenenfalls 7 700 bis 0,05, insbesondere 400 bis 2, vorzugsweise 20 bis 3 Gewichtsteilen Wasser (ebenfalls bezogen auf einen Gewichtsteil Komponente I, das heißt jeweils Gesamtmenge der Verbindungen I) verwendet. Diese Vermischung mit den Glycerinethern kann bei der Herstellung der entsprechenden Arzneimittel am Anfang erfolgen, aber gegebenenfalls auch in einem späteren Herstellungsstadium.

Beispiel für die Anwendungsweise der erfindungsgemäßen Verbindungen.

Die Wirkung wurde an 6 Personen mit Psoriasis vulgaris in stationärer Behandlung geprüft. Die erkrankten Personen hatten Hautrötungen, Verkrustungen auf der Haut, Schuppen auf der Haut (am ganzen Körper). Die Behandlung erfolgte topisch mit einer 6 %igen Lösung von Hexadecylphosphocholin in einem Gemisch aus Wasser, Glycerin-Propylether, Glycerin-Hexylether und Glycerin-Nonylether, im Verhältnis 2 : 1 : 1 : 2 (in Gewichtsteilen). Ein ml des vorstehend erwähnten Glycerin-Ether-Wassergemisches enthielt also 60 mg des Wirkstoffes Hexadecylphosphocholin (Kascade 60).

Diese Lösung wurde den Patienten zwei mal täglich (morgens und abends) während einer Woche auf die befallenen Hautstellen aufgetropft und unter leichtem Druck einmassiert (zum Beispiel mit einem Fingerling oder Handschuh aus Polyvinylchlorid (PVC). Nach einer Woche zeigte sich, daß sich bei allen Patienten die Schuppung der Schädigungen (Läsionen) gebessert und auch die Dicke der Läsionen abgenommen hat.

Beispiele:

Beispiel 1 (Lösung für topische Anwendung)

Hexadecylphosphocholin Lösung wird durch Auflösen von Hexadecylphosphocholin in einem als Kaskade 0 bezeichneten Lösungsmittel hergestellt.

Herstellung Kaskade 0

In einem geeigneten Behälter werden 1000 g Wasser, 1000 g Glycerin-1-n-propylether, 500 g Glycerin-1-n-hexylether und 500 g Glycerin-1-n-nonylether gemischt.

Herstellung der Lösung

In einem geeigneten Behälter werden ca. 2 Liter Kaskade 0 vorgelegt und 180 g Hexadecylphosphocholin unter Rühren gelöst. Schließlich wird mit Kaskade 0 auf 3 Liter aufgefüllt. Die Dichte der Lösung beträgt 1,003 g/ml bei 26°C.

Diese Lösung wird unter aseptischen Bedingungen in ein steriles Vorlagegefäß durch ein Membranfilter mit einer Porengröße von 0,2 µm filtriert und in sterile Tropfflaschen zu 10 ml abgefüllt. 1 ml der Lösung enthält 60 mg Hexadecylphosphocholin.

Beispiel 2 (Kapseln)

Hexadecylphosphocholin Hartgelatine-Kapseln 50 mg
250 g Hexadecylphosphocholin, 435,5 g Lactosemonohydrat DAB 9, 241,5 g mikrokristalline Cellulose DAB 9, 14 g Talkum DAB 9, 7 g hochdisperses Siliciumdioxid DAB 9 und 2 g Magnesiumstearat DAB 9 werden durch ein Sieb der Maschenweite 0,8 mm gegeben und anschließend in einem geeigneten Mischer 30 Min. homogenisiert.
Diese Kapselmasse wird auf einer Kapselfüllmaschine zu 190 mg in Hartgelatinesteckkapseln der Größe 2 dosiert.

1 Kapsel enthält 50 mg Hexadecylphosphocholin.

Beispiel 3 (Tabletten)

Hexadecylphosphocholin Tabletten 100 mg 300 g Hexadecylphosphocholin und 600 g Lactosemonohydrat DAB 9 werden durch ein 0,8 mm Sieb gegeben, in einem Wirbelschichtgranuliergerät gemischt und mit 180 g einer 10%igen Gelatinelösung granuliert.
Das Wirbelschichtgranulat, 82,8 g mikrokristalline Cellulose DAB 9, 120 g Maisstärke, 16,8 g Talkum und 2,4 g Magnesiumstearat werden durch ein Sieb 0,8 mm Maschinenweite gegeben. Diese Tablettenmasse wird auf einer geeigneten Tablettenpresse zu Tabletten von 380 mg Gewicht und einem Durchmesser von 10 mm verpreßt.
1 Tablette enthält 100 mg Hexadecylphosphocholin.

**Patentansprüche**

1. Verwendung von Verbindungen der allgemeinen Formel

$$R - Y - PO_2^{\theta} - X - R_1 \qquad I$$

oder einem physiologisch verträglichen Salz hiervon, wobei in der Formel I
R einen gesättigten oder ungesättigten Kohlenwasserstoffrest mit 12 bis 24 C-Atomen bedeutet, der auch halogensubstituiert sein kann, oder wobei R die Gruppe $-CH_2-CHR_3-CH_2-Z-R_4$ bedeutet und $R_3$ eine $C_1$-$C_6$-Alkoxygruppe oder ein $C_1$-$C_6$-Alkoxymethylgruppe ist, Z Sauerstoff oder Schwefel bedeutet und $R_4$ einen $C_1$-$C_{24}$-Alkylrest darstellt,
X ein Sauerstoffatom, NH oder $NR_2$ und Y ein Sauerstoffatom oder NH ist, $R_1$ eine $C_1$-$C_8$-Alkylgruppe ist, oder worin $R_1$ eine $C_2$-$C_8$-Alkylgruppe darstellt, die ungesättigt und/oder mit Halogen, Amino, $C_1$-$C_6$-Alkylamino, Di-$C_1$-$C_6$-alkylamino, Tri-$C_1$-$C_6$-alkylamino, Hydroxy, Carboxy, $C_3$-$C_8$-Cycloalkyl oder Phenyl substituiert ist, und worin $R_1$ außerdem auch 2-tert.-Butyloxycarbonylaminoethyl, 2-tert.-Butyloxycarbonylethyl, 2,3-Isopropylidendioxy-propyl-(1), 2,3-Dibenzyloxy-propyl-(1), 1,3-Dibenzyloxy-propyl-(2) oder N-$C_1$-$C_6$-Alkylamino-$C_2$-$C_6$-alkyl bedeuten kann, wenn X ein Sauerstoffatom ist, und worin $R_1$ außerdem auch 2,3-Dihydroxypropyl-(1) bedeuten kann, wenn X die NH-Gruppe ist,
und $R_2$ eine 2,3-Dihydroxypropyl-(1)-gruppe, eine $C_1$-$C_8$-Alkylgruppe oder eine $C_2$-$C_8$-Alkylgruppe, die ungesättigt und/oder mit Halogen, Amino, $C_1$-$C_6$-Alkylamino, Di-$C_1$-$C_6$-alkylamino, Tri-$C_1$-$C_6$-alkylamino, Hydroxy, Carboxy, $C_3$-$C_8$-Cycloalkyl oder Phenyl substituiert ist, darstellt,
für die Herstellung eines Arzneimittels zur Bekämpfung von Psoriasis-Erkrankungen.

2. Mittel zur topischen Behandlung von Psoriasis-Erkrankungen, enthaltend als Wirkstoff mindestens eine Verbindung der allgemeinen Formel

$$R - Y - PO_2^{\theta} - X - R_1 \qquad I$$

oder ein physiologisch verträgliches Salz hiervon, wobei in der Formel I
R einen gesättigten oder ungesättigten Kohlenwasserstoffrest mit 12 bis 24 C-Atomen bedeutet, der auch halogensubstituiert sein kann, oder wobei R die Gruppe $-CH_2-CHR_3-CH_2-Z-R_4$ bedeutet und $R_3$ eine $C_1$-$C_6$-Alkoxygruppe oder ein $C_1$-$C_6$-Alkoxymethylgruppe ist, Z Sauerstoff oder Schwefel bedeutet und $R_4$ einen $C_1$-$C_{24}$-Alkylrest darstellt,
X ein Sauerstoffatom, NH oder $NR_2$ und Y ein Sauerstoffatom oder NH ist, $R_1$ eine $C_1$-$C_8$-Alkylgruppe ist, oder worin $R_1$ eine $C_2$-$C_8$-Alkylgruppe darstellt, die ungesättigt und/oder mit Halogen, Amino, $C_1$-$C_6$-Alkylamino, Di-$C_1$-$C_6$-alkylamino, Tri-$C_1$-$C_6$-alkylamino, Hydroxy, Carboxy, $C_3$-$C_8$-Cycloalkyl oder Phenyl substituiert ist, und worin $R_1$ außerdem auch 2-tert.-Butyloxycarbonylaminoethyl, 2-tert.-Butyloxycarbonylethyl, 2,3-Isopropylidendioxy-propyl-(1), 2,3-Dibenzyloxy-propyl-(1), 1,3-Dibenzyloxy-propyl-(2) oder N-$C_1$-$C_6$-Alkylamino-$C_2$-$C_6$-alkyl bedeuten kann, wenn X ein Sauerstoffatom ist, und worin $R_1$ außerdem auch 2,3-Dihydroxypropyl-(1) bedeuten kann, wenn X die NH-Gruppe ist,
und $R_2$ eine 2,3-Dihydroxypropyl-(1)-gruppe, eine $C_1$-$C_8$-Alkylgruppe oder eine $C_2$-$C_8$-Alkylgruppe, die ungesättigt und/oder mit Halogen, Amino, $C_1$-$C_6$-Alkylamino, Di-$C_1$-$C_6$-alkylamino, Tri-$C_1$-$C_6$-alkylamino, Hydroxy, Carboxy, $C_3$-$C_8$-Cycloalkyl oder Phenyl substituiert ist, darstellt.

3. Mittel nach Anspruch 2,
dadurch gekennzeichnet, daß es übliche pharmazeutische Träger-, Hilfs- und/oder Verdünnungsmittel enthält.

4. Mittel nach einem oder mehreren der vorangegangenen Ansprüche, dadurch gekennzeichnet,

daß es als Wirkstoff eine Verbindung gemäß Anspruch 1 sowie ein Alkylglycerin der allgemeinen Formel II

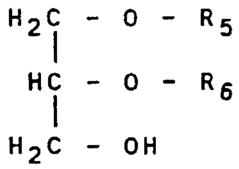

II

enthält, wobei in der Formel II einer der Reste $R_5$ und $R_6$ eine Alkylgruppe mit 2 bis 12 C-Atomen und der andere Rest ein H-Atom bedeutet, sowie gegebenenfalls weitere übliche pharmazeutische Zusatz-, Träger- und/oder Verdünnungsstoffe.

**5.** Verfahren zur Herstellung eines Arzneimittels zur Bekämpfung von Psoriasis-Erkrankungen,
dadurch gekennzeichnet, daß mindestens eine Verbindung der allgemeinen Formel

$$R - Y - PO_2^{\ominus} - X - R_1 \qquad I$$

oder ein physiologisch verträgliches Salz hiervon, wobei in der Formel I
R einen gesättigten oder ungesättigten Kohlenwasserstoffrest mit 12 bis 24 C-Atomen bedeutet, der auch halogensubstituiert sein kann, oder wobei R die Gruppe $-CH_2-CHR_3-CH_2-Z-R_4$ bedeutet und $R_3$ eine $C_1$-$C_6$-Alkoxygruppe oder ein $C_1$-$C_6$-Alkoxymethylgruppe ist, Z Sauerstoff oder Schwefel bedeutet und $R_4$ einen $C_1$-$C_{24}$-Alkylrest darstellt,
X ein Sauerstoffatom, NH oder $NR_2$ und Y ein Sauerstoffatom oder NH ist, $R_1$ eine $C_1$-$C_8$-Alkylgruppe ist, oder worin $R_1$ eine $C_2$-$C_8$-Alkylgruppe darstellt, die ungesättigt und/oder mit Halogen, Amino, $C_1$-$C_6$-Alkylamino, Di-$C_1$-$C_6$-alkylamino, Tri-$C_1$-$C_6$-alkylamino, Hydroxy, Carboxy, $C_3$-$C_8$-Cycloalkyl oder Phenyl substituiert ist, und worin $R_1$ außerdem auch 2-tert.-Butyloxycarbonylaminoethyl, 2-tert.-Butyloxycarbonylethyl, 2,3-Isopropylidendioxy-propyl-(1), 2,3-Dibenzyloxy-propyl-(1), 1,3-Dibenzyloxy-propyl-(2) oder N-$C_1$-$C_6$-Alkylamino-$C_2$-$C_6$-alkyl bedeuten kann, wenn X ein Sauerstoffatom ist, und worin $R_1$ außerdem auch 2,3-Dihydroxypropyl-(1) bedeuten kann, wenn X die NH-Gruppe ist,
und $R_2$ eine 2,3-Dihydroxypropyl-(1)-gruppe, eine $C_1$-$C_8$-Alkylgruppe oder eine $C_2$-$C_8$-Alkylgruppe, die ungesättigt und/oder mit Halogen, Amino, $C_1$-$C_6$-Alkylamino, Di-$C_1$-$C_6$-alkylamino, Tri-$C_1$-$C_6$-alkylamino, Hydroxy, Carboxy, $C_3$-$C_8$-Cycloalkyl oder Phenyl substituiert ist, darstellt,
mit üblichen pharmazeutischen Träger-, Hilfs- und/oder Verdünnungsmitteln zu einem Arzneimittel für die Behandlung von Psoriasis-Erkrankungen verarbeitet wird.

**6.** Verfahren zur Herstellung eines Arzneimittels gemäß einem oder mehreren der vorangegangenen Verfahrensansprüche,
dadurch gekennzeichnet,
daß man bei der Herstellung zusätzlich noch ein Alkylglycerin der Formel II

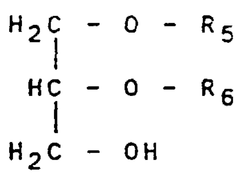

II

in der einer der Reste $R_5$ und $R_6$ eine Alkylgruppe mit 2 bis 12 C-Atomen und der andere Rest ein H-Atom bedeutet, oder ein Gemisch solcher Alkylglycerine sowie gegebenenfalls Wasser verwendet, wobei man 10 000 bis 7 Gewichtsteile Alkylglycerin der Formel II beziehungsweise eines entsprechenden Alkylglyceringemisches und gegebenenfalls 7 700 bis 0,05 Gewichtsteile Wasser, jeweils bezogen auf einen Gewichtsteil der Komponente I, verwendet.

**Geänderte Patentansprüche gemäss Regel 86(2) EPÜ.**

1. Verwendung von Verbindungen der allgemeinen Formel

$$R - Y - PO_2^{\theta} - X - R_1 \qquad I$$

oder einem physiologisch verträglichen Salz hiervon, wobei in der Formel I
R einen gesättigten oder ungesättigten Kohlenwasserstoffrest mit 12 bis 24 C-Atomen bedeutet, der auch halogensubstituiert sein kann, oder wobei R die Gruppe $-CH_2-CHR_3-CH_2-Z-R_4$ bedeutet und $R_3$ eine $C_1$-$C_6$-Alkoxygruppe oder ein $C_1$-$C_6$-Alkoxymethylgruppe ist, Z Sauerstoff oder Schwefel bedeutet und $R_4$ einen $C_1$-$C_{24}$-Alkylrest darstellt,
X ein Sauerstoffatom, NH oder $NR_2$ und Y ein Sauerstoffatom oder NH ist, $R_1$ eine $C_1$-$C_8$-Alkylgruppe ist, oder worin $R_1$ eine $C_2$-$C_8$-Alkylgruppe darstellt, die ungesättigt und/oder mit Halogen, Amino, $C_1$-$C_6$-Alkylamino, Di-$C_1$-$C_6$-alkylamino, Tri-$C_1$-$C_6$-alkylamino, Hydroxy, Carboxy, $C_3$-$C_8$-Cycloalkyl oder Phenyl substituiert ist, und worin $R_1$ außerdem auch 2-tert.-Butyloxycarbonylaminoethyl, 2-tert.-Butyloxycarbonylethyl, 2,3-Isopropylidendioxy-propyl-(1), 2,3-Dibenzyloxy-propyl-(1), 1,3-Dibenzyloxy-propyl-(2) oder N-$C_1$-$C_6$-Alkylamino-$C_2$-$C_6$-alkyl bedeuten kann, wenn X ein Sauerstoffatom ist, und worin $R_1$ außerdem auch 2,3-Dihydroxypropyl-(1) bedeuten kann, wenn X die NH-Gruppe ist,
und $R_2$ eine 2,3-Dihydroxypropyl-(1)-gruppe, eine $C_1$-$C_8$-Alkylgruppe oder eine $C_2$-$C_8$-Alkylgruppe, die ungesättigt und/oder mit Halogen, Amino, $C_1$-$C_6$-Alkylamino, Di-$C_1$-$C_6$-alkylamino, Tri-$C_1$-$C_6$-alkylamino, Hydroxy, Carboxy, $C_3$-$C_8$-Cycloalkyl oder Phenyl substituiert ist, darstellt,
für die Herstellung eines Arzneimittels zur Bekämpfung von mit Hyperkeratosen einhergehende Hauterkrankungen.

2. Mittel zur topischen Behandlung von mit Hyperkeratosen einhergehende Hauterkrankungen, enthaltend als Wirstoff mindestens eine Verbindung der allgemeinen Formel

$$R - Y - PO_2^{\theta} - X - R_1 \qquad I$$

oder ein physiologisch verträgliches Salz hiervon, wobei in der Formel I
R einen gesättigten oder ungesättigten Kohlenwasserstoffrest mit 12 bis 24 C-Atomen bedeutet, der auch halogensubstituiert sein kann, oder wobei R die Gruppe $-CH_2-CHR_3-CH_2-Z-R_4$ bedeutet und $R_3$ eine $C_1$-$C_6$-Alkoxygruppe oder ein $C_1$-$C_6$-Alkoxymethylgruppe ist, Z Sauerstoff oder Schwefel bedeutet und $R_4$ einen $C_1$-$C_{24}$-Alkylrest darstellt,
X ein Sauerstoffatom, NH oder $NR_2$ und Y ein Sauerstoffatom oder NH ist, $R_1$ eine $C_1$-$C_8$-Alkylgruppe ist, oder worin $R_1$ eine $C_2$-$C_8$-Alkylgruppe darstellt, die ungesättigt und/oder mit Halogen, Amino, $C_1$-$C_6$-Alkylamino, Di-$C_1$-$C_6$-alkylamino, Tri-$C_1$-$C_6$-alkylamino, Hydroxy, Carboxy, $C_3$-$C_8$-Cycloalkyl oder Phenyl substituiert ist, und worin $R_1$ außerdem auch 2-tert.-Butyloxycarbonylaminoethyl, 2-tert.-Butylexycarbonylethyl, 2,3-Isopropylidendioxy-propyl-(1), 2,3-Dibenzyloxy-propyl-(1), 1,3-Dibenzyloxy-propyl-(2) oder N-$C_1$-$C_6$-Alkylamino-$C_2$-$C_6$-alkyl bedeuten kann, wenn X ein Sauerstoffatom ist, und worin $R_1$ außerdem auch 2,3-Dihydroxypropyl-(1) bedeuten kann, wenn X die NH-Gruppe ist,
und $R_2$ eine 2,3-Dihydroxypropyl-(1)-gruppe, eine $C_1$-$C_8$-Alkylgruppe oder eine $C_2$-$C_8$-Alkylgruppe, die ungesättigt und/oder mit Halogen, Amino, $C_1$-$C_6$-Alkylamino, Di-$C_1$-$C_6$-alkylamino, Tri-$C_1$-$C_6$-alkylamino, Hydroxy, Carboxy, $C_3$-$C_8$-Cycloalkyl oder Phenyl substituiert ist, darstellt.

3. Mittel nach Anspruch 2
dadurch gekennzeichnet, daß es übliche pharmazeutische Träger-, Hilfs- und/oder Verdünnungsmittel enthält.

4. Mittel nach einem oder mehreren der vorangegangenen Ansprüche, dadurch gekennzeichnet,
daß es als Wirkstoff eine Verbindung gemäß Anspruch 1 sowie ein Alkylglycerin der allgemeinen Formel II

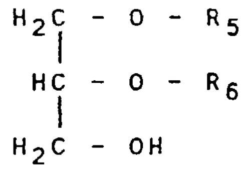

II

enthält, wobei in der Formel II einer der Reste $R_5$ und $R_6$ eine Alkylgruppe mit 2 bis 12 C-Atomen und der andere Rest ein H-Atom bedeutet, sowie gegebenenfalls weitere übliche pharmazeutische Zusatz-, Träger- und/oder Verdünnungsstoffe.

**5.** Verfahren zur Herstellung eines Arzneimittels zur Bekämpfung von mit Hyperkeratosen einhergehende Hauterkrankungen, dadurch gekennzeichnet, daß mindestens eine Verbindung der allgemeinen Formel

$$R - Y - PO_2^{\theta} - X - R_1 \qquad I$$

oder ein physiologisch verträgliches Salz hiervon, wobei in der Formel I
R einen gesättigten oder ungesättigten Kohlenwasserstoffrest mit 12 bis 24 C-Atomen bedeutet, der auch halogensubstituiert sein kann, oder wobei R die Gruppe $-CH_2-CHR_3-CH_2-Z-R_4$ bedeutet und $R_3$ eine $C_1$-$C_6$-Alkoxygruppe oder ein $C_1$-$C_6$-Alkoxymethylgruppe ist, Z Sauerstoff oder Schwefel bedeutet und $R_4$ einen $C_1$-$C_{24}$-Alkylrest darstellt,
X ein Sauerstoffatom, NH oder $NR_2$ und Y ein Sauerstoffatem oder NH ist, $R_1$ eine $C_1$-$C_8$-Alkylgruppe ist, oder worin $R_1$ eine $C_2$-$C_8$-Alkylgruppe darstellt, die ungesättigt und/oder mit Halogen, Amino, $C_1$-$C_6$-Alkylamino, Di-$C_1$-$C_6$-alkylamino, Tri-$C_1$-$C_6$-alkylamino, Hydroxy, Carboxy, $C_3$-$C_8$-Cycloalkyl oder Phenyl substituiert ist, und worin $R_1$ außerdem auch 2-tert.-Butyloxycarbenylaminoethyl, 2-tert.-Butyloxycarbonylethyl, 2,3-Isopropylidendioxy-propyl-(1), 2,3-Dibenzyloxy-propyl-(1), 1,3-Dibenzyloxy-propyl-(2) oder N-$C_1$-$C_6$-Alkylamino-$C_2$-$C_6$-alkyl bedeuten kann, wenn X ein Sauerstoffatom ist, und worin $R_1$ außerdem auch 2,3-Dihydroxypropyl-(1) bedeuten kann, wenn X die NH-Gruppe ist,
und $R_2$ eine 2,3-Dihydroxypropyl-(1)-gruppe, eine $C_1$-$C_8$-Alkylgruppe oder eine $C_2$-$C_8$-Alkylgruppe, die ungesättigt und/oder mit Halogen, Amino, $C_1$-$C_6$-Alkylamino, Di-$C_1$-$C_6$-alkylamino, Tri-$C_1$-$C_6$-alkylamino, Hydroxy, Carboxy, $C_3$-$C_8$-Cycloalkyl oder Phenyl substituiert ist, darstellt,
mit üblichen pharmazeutischen Träger-, Hilfs- und/oder Verdünnungsmitteln zu einem Arzneimittel für die Behandlung von mit Hyperkeratosen einhergehende Hauterkrankungen verarbeitet wird.

**6.** Verfahren zur Herstellung eines Arzneimittels gemäß einem oder mehreren der vorangegangenen Verfahrensansprüche,
dadurch gekennzeichnet,
daß man bei der Herstellung zusätzlich noch ein Alkylglycerin der Formel II

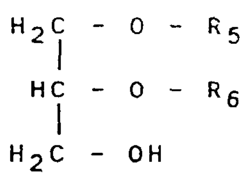

II

in der einer der Reste $R_5$ und $R_6$ eine Alkylgruppe mit 2 bis 12 C-Atomen und der andere Rest ein H-Atom bedeutet, oder ein Gemisch solcher Alkylglycerine sowie gegebenenfalls Wasser verwendet, wobei man 10 000 bis 7 Gewichtstelle Alkylglycerin der Formel II beziehungsweise eines entsprechenden Alkylglyceringemisches und gegebenenfalls 7 700 bis 0,05 Gewichtsteile Wasser, jeweils bezogen auf einen Gewichtsteil der Komponente I, verwendet.

Europäisches Patentamt

# EUROPÄISCHER RECHERCHENBERICHT

Nummer der Anmeldung
EP 98 11 2091

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | EP 0 208 961 A (MERCK & CO. INC.)<br>21. Januar 1987<br>* das ganze Dokument * | 1-3,5 | A61K31/66<br>//(A61K31/66,<br>31:08,47:08) |
| X,P | DE 38 29 899 A (REUTTER WERNER)<br>8. März 1990<br>* das ganze Dokument * | 1-3,5 | |
| X | WO 87 03478 A (MAX-PLAMCK GESELLSCHAFT ZUR FÖRDERUNG DER WISSENSCHAFTEN E.V.)<br>18. Juni 1987<br>* Zusammenfassung *<br>* Seite 2 - Seite 3 *<br>* Seite 21 - Seite 27 * | 2-6 | |
| X | UNGER ET AL.: "Hexadecylphosphocholine (D 18506) in the topical treatment of skin"<br>ONKOLOGIE,<br>Bd. 11, Nr. 6, Dezember 1988, Seiten 295-296, XP002094714<br>* das ganze Dokument * | 2-6 | |
| A | | 1 | |

| | RECHERCHIERTE SACHGEBIETE (Int.Cl.6) |
|---|---|
| | A61K |

Der vorliegende Recherchenbericht wurde für alle Patentansprüche erstellt

| Recherchenort | Abschlußdatum der Recherche | Prüfer |
|---|---|---|
| DEN HAAG | 25. Februar 1999 | Gac, G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie,übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 98 11 2091

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-02-1999

| Im Recherchenbericht angeführtes Patentdokument | Datum der Veröffentlichung | Mitglied(er) der Patentfamilie | Datum der Veröffentlichung |
|---|---|---|---|
| EP 208961 A | 21-01-1987 | US 4761404 A | 02-08-1988 |
| | | JP 62012754 A | 21-01-1987 |
| DE 3829899 A | 08-03-1990 | DE 4006564 A | 05-09-1991 |
| | | DE 4006563 A | 05-09-1991 |
| WO 8703478 A | 18-06-1987 | CA 1338627 A | 01-10-1996 |
| | | CA 1280369 A | 19-02-1991 |
| | | DD 281961 A | 29-08-1990 |
| | | DD 259405 A | 24-08-1988 |
| | | DD 285293 A | 12-12-1990 |
| | | DE 3641379 A | 03-09-1987 |
| | | DE 3641491 A | 17-09-1987 |
| | | DE 3685214 D | 11-06-1992 |
| | | DK 393187 A | 28-07-1987 |
| | | DK 398587 A | 30-07-1987 |
| | | WO 8703480 A | 18-06-1987 |
| | | EP 0225608 A | 16-06-1987 |
| | | EP 0230575 A | 05-08-1987 |
| | | EP 0248047 A | 09-12-1987 |
| | | EP 0248062 A | 09-12-1987 |
| | | FI 873363 A | 03-08-1987 |
| | | FI 873364 A | 03-08-1987 |
| | | GR 3002487 T | 30-12-1992 |
| | | GR 3005168 T | 24-05-1993 |
| | | IE 59777 B | 06-04-1994 |
| | | IE 59778 B | 06-04-1994 |
| | | JP 8059677 A | 05-03-1996 |
| | | JP 2697736 B | 14-01-1998 |
| | | JP 63501874 T | 28-07-1988 |
| | | JP 6037393 B | 18-05-1994 |
| | | JP 63501719 T | 14-07-1988 |
| | | NO 174877 B | 18-04-1994 |
| | | NO 175620 B | 01-08-1994 |
| | | PT 83872 B | 17-01-1989 |
| | | PT 83873 B | 17-01-1989 |
| | | US 4837023 A | 06-06-1989 |
| | | US 5049552 A | 17-09-1991 |

EPO FORM P0461

Für nähere Einzelheiten zu diesem Anhang : siehe Amtsblatt des Europäischen Patentamts, Nr.12/82